# EUROPEAN PATENT APPLICATION

(11) **EP 2 005 948 A2**
(43) Date of publication of application: **24.12.2008**
(21) Application number: 06799621.5
(22) Date of filing: 14.03.2006
(51) Int. Cl.: A61K 31/03, A61K 31/02, A61K 9/107, A61K 9/06, A61P 7/08, A61K 8/04, A61Q 19/00

(54) **PERFLUOROCARBON GAS TRANSFERRING EMULSION FOR MEDICO-BIOLOGICAL USE, THE COMPOSITION AND THE PRODUCTION METHOD THEREOF A MEDICINAL AGENT**

(71) Applicant: Vorobyev, Sergey Ivanovich, Moscow 119571 (RU)
(72) Inventor: Vorobyev, Sergey Ivanovich, Moscow 119571 (RU)
(74) Representative: Müller Fottner Steinecke
(86) International application number: PCT/RU2006/000113
(87) International publication number: WO 2007/105978

(57) **Abstract**

The invention belongs to medicobiological industry and refers to the composition, method of production and use of the means, based on perfluorocarbon emulsions, intended as the blood substitute means and other remedies. The essence of invention consists in creation of method and composition made from the mixture of two perfluorocarbons: fast eliminated (Sv-syu) and (or) slowly eliminated (Sc-sp), as perfluorodecaline (PFD) / perfluoromethyl-cyclo-hexylpiperidine (PFMCP), or PFD/perfluorotributylamine (PFTBA), or perfluorooctylbromide (PFOB)/PFMCP, or PFOB/PFTBA, or PFOB/PFD, or PFMCP/PFTBA in ratio from 1/1 to 10/10, respectively; or a mixture of three perfluorocarbons: two are fast eliminated (C₈-C₁₀) and one is slowly eliminated (C₁₁-C₁₂), or of one fast eliminated (C₈-C₁₀) and two slowly eliminated (C₁₁-C₁₂), as PFOB/PFD/PFMCP, or PFOB/PFD/PFTBA, or PFOB/PFMCT/PFTBA, or PFD/PFMCP/PFTBA in ratio from 1/1/1 to 10/10/10, respectively; or a mixture of four perfluorocarbons: two of them are fast eliminated (C₈-C₁₀) and two slowly eliminated (C₁₁-C₁₂) PFOB/PFD/PFMCP/PFTBA in ratio from 1/1/1/1 to 10/10/10/10, respectively; a mixture of perfluorocarbons in emulsion from 1% (0,5 vol.%) to 100% (50 vol. %) with average particle size 30-80 nm, is emulsified by proxanol-268 from 0,2% to 20% with molecular weight 6-12 thousand Da, has acceptable electrolytic solution.

## Description

The invention is referred to medicinal and biological industry and concerns to composition, method of production and application of agents based on perfluorocarbonic gas-transferring emulsions, meant for use as synthetic perfluorocarbonic blood substitutes and agents for different diseases treatment as perfusions, X-rays contrast and cosmetic remedies.

### Previous technical level

### Perfluorocarbonic emulsions composition

Perfluorocarbonic emulsions represent dispersion of the liquid phase (Perfluorocarbons), distributed in other liquid (water)-dispersion medium. In accordance with the aggregative state such dispersion system has designation Liquid/Liquid (L/L). In the perfluorocarbonic emulsion water, polar liquid, is the dispersion medium. Perfluorocarbonic emulsions are direct emulsions. Most of dispersion systems with liquid dispersion medium are aggregatively unstable, i.e. lyophobic, and perfluorocarbonic emulsions are one of them, so thermodynamically nonequilibrium systems.

Dispersion's properties depend on dispersion phase particles quantity. If there are not many particles, the contact between them is insignificant and particles can move ones upon others, and such dispersions are free-dispersive. Perfluorocarbonic emulsions are free-dispersive systems having 1,53*10¹⁸ particles per 11 of 20 % emulsion.

In most part of cases in creation of highly dispersive systems a work consumption as mechanical energy is required. Dispersion method is the most reasonable for obtaining nano-emulsions, it is based on the mechanical method of overcoming of intermolecular forces. Dispersion with high pressure homogenizers gives liquid perfluorocarbons size reduction to sub-micron size. In this case the dispersion increases dramatically and a dispersion system having developed specific surface area forms. So, in the process of emulsion forming, in the process of dispersion of two liquids the phase interface does not decrease, but increases. Dispersion phase defragmentation gives dispersion systems special properties, related to the size of phase interface between dispersion phase and dispersion medium.

Homogenization effectiveness, as a rule, is determined by size of medium diameter of the particles of obtained dispersion phase. Average diameter of perfluorocarbons emulsion particles depends on dispersion cycles quantity: D av. = f (N).as per their dispersion, perfluorocarbonic emulsions can be conditionally separated to two general types - monodisperse and polydisperse emulsions. Polydisperse emulsions size is in the range between 0.1 µm (100 nm) and 10 µm and more, which corresponds to the size of medium and coarsely dispersed systems. Monodisperse system's size is in the range between 1 nm (10⁻⁹ m) and 100 nm, which corresponds to the size of highly disperse systems.

It is necessary to give a colloid-chemical definition for perfluorocarbonic emulsions of medicinal and biological application - *they are direct, concentrated, highly- and freely-disperse, heterogenic thermodynamically unstable colloid systems, having considerate free energy and huge gas-exchange surface (phase interface), in which disperse phase, insoluble monodisperse nano-particles of perfluorocarbons, is covered by surface active layer of emulsifier and is in disperse water structured medium in suspension state.*

In this state the substance, nano-particles of perfluoroemulsion (10⁻⁸ m), will have particular properties, because this state is special intermediate state of substance between associated compound and ultradisperse (colloid) state. In accordance with structural properties, the area of sizes is in the scope between 10 and 30 nm (10⁻⁸ m), is critical.

In formulations for medicinal and biological use based on perfluorocarbonic emulsions, as a rule, some types of perfluorocarbons are used simultaneously. One of them is from group (C₈-C₁₀), and contains, for example, perfluorodecaline (PFD) C₁₀F₁₈ or perfluorotripropylamine (PFTPA) C₉F₂₁N or perfluorooctylbromide (PFOB) C₈F₁₇Br, second one - of the group (C₁₁-C₁₂), which contains, for example, perfluoromethylcyclohexylpiperidine (PFMCP) C₁₂F₂₃N or perfluorotributylamine (PFTBA) C₁₂F₂₇N. These perfluorocarbons dissolve 40 % vol. of oxygen (in case if pO₂ = 760 mm Hg) and 150 - 190 % vol. of carbonic acid gas (in pCO₂ = 760 mm Hg), because of that they are used as a principal compound - gas-carrier in creation of synthetic blood substitutes. However, perfluorocarbons are insoluble in water and other liquids, and because of this they can be used in form of emulsion with defined size of perfluorocarbonic particles, covered by the layer of emulsifier (proxanol or phospholipide) only. The smaller is the particle size, the better, because the emulsion is introduced intravenously and in case of big size particles emulsion formulations can produce embolia (congestion) of vessels and severe adverse reactions on presence of coarsely dispersed emulsion particles. First type substances rapidly (during one month) are eliminated from the organism but do not provide necessary emulsion stability, second type substances, in contrary, give high stability of emulsion, which allows to keep them without freezing, but they persist in the organism during some years.

There are known emulsion formulations which contain, for example, perfluorodecaline (PFD) and perfluorotripropylamine (PFTPA), emulsifying agents, for example, co-polymer of polyoxiethylenepropylene (pluronic F-68, domestic analogue - proxanol 268), egg's yolk phospholipides or soya phospholipides and water (certificate of authorship USSR No. 797546, published in the bulletin "Discoveries, inventions...", 1981, No. 2). In accordance with this composition perfluorocarbons concentration is 24% in physiologically appropriate water medium. Disadvantages of this composition in the signed invention are that the composition of emulsion has quite coarsely dispersed particles by their size and can't be stored in defreezed state, and presence of severe adverse reactions and it is impossible to sterilize it.

In composition of other 20 % emulsion, prepared for medicinal use on the base of PFD and PFTPA - Fluosol-DA 20 %, Japan firm production, proxanol and egg yolk phospholipides were used as emulsifiers. However, average particle diameter in composition of this emulsion was big, too, because in high temperature during emulsifying and pasteurization a growth of particle size takes place. Moreover particles of perfluorocarbons used in this suspension grow quite fast even at the room temperature (Mitsuno T. et al., "Intake and retention of perfluorochemical substance of Fluosol-DA in res human", Proceedings of 5 Sympos. On Oxygen-Carrying Colloidal Blood Substitutes, Mainz, March, 1981, p. 220). This composition of emulsion can be kept freezed only, because after 8-12 hours of storage at the room temperature growth of particle size happens and, so, clinical application becomes impossible. Moreover, the lacks of this composition in signed invention are severe adverse reactions.

It is known the composition for medicinal purposes (patent RF No. 2070033, published in the bull. "Discoveries and inventions..." in 1996, No. 34), which includes perfluorodecaline or perfluorooctylbromide and perfluoromethylcyclohexylpiperidine in the ratio from 20 to 40 %. Disadvantages of this composition are adverse reactions and low perfluorocarbons concentration in the emulsion - not higher than 40%.

The composition of perfluorocarbon emulsion for medical and biological purposes (patent of RF No. 2122404, published in bull. "Discoveries, inventions...") is close to the present composition. There are perfluorodecaline, perfluoromethylcyclohexylpiperidine, perfluorooctylbromide, perfluorotributylamide, emulsified with proxanol-268.

The disadvantages of this composition are adverse reactions, which frequency is about 20 % and low perfluorocarbons concentration in the emulsion - not higher 40 %.

The closest to the present composition is the composition of perfluorocarbon emulsion for medical and biological purposes (patent of RF No. 216292, published in bull. "Discoveries, inventions..." 10.02.2002, No. 4). There are perfluorodecaline, perfluoromethylcyclohexylpiperidine, perfluorooctylbromide, perfluorotributylamide in the concentrations of 1-20 %, emulsified with 0.4-4.8% proxanol-268. The disadvantages of this composition are low perfluorocarbons concentration in the emulsion - not higher 20 % what limits it's application area.

The closest to the present composition is the composition of perfluorocarbon emulsion for medical and biological purposes (patent of RF No. 2199311, published in bull. "Discoveries, inventions..." 27.02.2003, No. 6). There are perfluorodecaline, perfluoromethylcyclohexylpiperidine in the concentrations of 1-30 %, emulsified with 0.2-6% proxanol. The disadvantages of this composition are low perfluorocarbons concentration in the emulsion - not higher 30 % what limits it's application area.

### Perfluorocarbon emulsions production method

The more common methods of perfluorocarbons emulsions production are based on methods using ultrasound and homogenization on disintegrators under high pressure. For industrial emulsion production the homogenization method is preferred, i.e. it allows to obtain emulsions in big quantities and with better physical and chemical characteristics, for example, better particle size distribution (Jean G. Riess and Maurice Le Blanc. Preparation of perfluoro-chemical emulsions for biochemical use: principles, materials and methods. Ellis Horwood Series in Biomedicine, VCH, Blood Substitutes, Preparation, Physiology and Medical Applications. 1991. Ch. 5, p.p. 113-115).

A method of preparation perfluorocarbons emulsions, containing, for example, perfluorodecaline and perfluorotripropylamine, emulsifying agents, for example, polyoxiethylene-propylene co-polymer (pluronic F-68, domestic analogue - proxanol-268), egg yolk phospholipides or soybean phospholipides and water (Patent of USSR No. 797546, published in bull. "Discoveries, inventions..." 1981, No. 2). As per this method, the initial perfluorocarbon emulsion should be prepared with mixing of compounds in physiologically appropriate aqueous medium by homogeneous mixer or propeller mixer, after that emulsify essential emulsion by injecting in the high pressure homogenizer from 100 to 500 atm. and temperature + 55 °C. Perfluorocarbon mix is emulsified through one extrusion device of the homogenizer till 12 times (emulsification cycles). The disadvantages of this invention are a long time of perfluorocarbon suspension production by this method (12 emulsification cycles), low concentration (not higher 24%) and it can not be stored in defreezed state and can't be sterilized.

It is known the method of perfluorocarbon emulsions production for medicinal application (patent of RF No. 2070033, published in the bull. "Discoveries, inventions..." 1996, No. 34), close to the present method, in which 12-times (12 cycles) emulgation of perfluorodecaline or perfluorooctylbromide with perfluoromethylcyclohexylpiperidine, is realized in 3 vessels. The disadvantages of this described way of perfluorocarbon emulsion production are a long time of perfluorocarbon suspension production by this method (12 emulsification cycles), the impossibility to prepare the high concentration emulsions more than 40 % and that it can't be sterilized.

It is known the method of perfluorocarbon compositions preparation for medicinal application (patent of RF No. 2122404, published in bull. "Discoveries, inventions..." 27.11.1998, No. 33), close to applied method, in which 40% emulsion is obtained by 9-times (9 cycles) of emulsification and jet-dripping passing of multicompound mix of some perfluorocarbons. The disadvantages of this described way of perfluorocarbon emulsion production are a long time of perfluorocarbon emulsifying - 9 cycles, the impossibility to prepare the high concentration emulsions (more than 40 %) and the impossibility to sterilize these emulsions.

The closest to the present perfluorocarbon emulsion preparation method is the method of preparation of perfluorocarbon emulsion for medical and biological purposes (patent of RF No. 2200544, published in bull. "Discoveries, inventions..." 20.03.2003, No. 8). This method is based on 9-times (9 cycles) of emulsification and jet-dripping passing of multicompound mix of some perfluorocarbons, consisting of the mix of some perfluorocarbons, through the proxanol-268 solution and one extrusion device, and obtaining of 40% emulsion. The lack of this described way of perfluorocarbon emulsion production is the impossibility to obtain high concentrated emulsions (more than 40 %) because of presence of one extrusion device of homogenizer, long time of emulsifying (for example, 10 1 of 10% vol. emulsion can be produced within 3-4 conditional or relative hours), what is due to the presence of one only homogenizer's extrusion device in which emulsion preparation passes. This results in long term presence of operator in the sterile box and possible de-sterilization (semination) of emulsion.

### Disclosure of invention.

The first object of the proposed invention is creation of the formulation of multicompound, highly concentrated, having high stability perfluorocarbon emulsions, with different physical and chemical properties with decreased frequency of adverse reactions and with usage of physiologically acceptable and simple electrolyte solutions.

The second object of the proposed invention is creation of method of obtaining of the method of preparation of multicompound, highly concentrated, highly stable, sterilized perfluorocarbon emulsions having nano-sized average diameter of particles and acceleration of the total process of production of perfluorocarbon suspensions, that is necessary for the production of perfluoroemulsions in semicommercial scales.

The third object of the proposed invention is application of the developed agent based of the perfluorocarbon gas transferring emulsion in different medical and biological spheres.

The solution of the first determined objective is that the known composition on the base of perfluorocarbon gas transferring emulsion for medical and biological purposes, including: perfluorocarbons, emulsifying agent and electrolyte solution, as per invention, contains:
the mix of two perfluorocarbons: rapidly eliminated (C₈-C₁₀) and/or slowly eliminated (C₁₁-C₁₂), such as perfluorodecaline (PFD)/perfluoromethylcyclohexylpiperidine (PFMCP), or PFD/perfluorotributylamine (PFTBA), or perfluorooctylbromide (PFOB)/PFMCP, or PFOB/PFTBA, or PFOB/PFD, or PFMCP/PFTBA in the ratio from 1/1 to 10/10, respectively; or the mix of three perfluorocarbons: two rapidly eliminated (C₈-C₁₀) and one slowly eliminated (C₁₁-C₁₂), or one rapidly eliminated (C₈-C₁₀) and two slowly eliminated (C₁₁-C₁₂), such as PFOB/PFD/PFMCP, or PFOB/PFD/PFTBA, or PFOB/PFMCP/PFTBA, or PFD/PFMCP/PFTBA in the ratio from 1/1/1 to 10/10/10, respectively; or the mix of four perfluorocarbons: two rapidly eliminated (C₈-C₁₀) and two slowly eliminated (C₁₁-C₁₂), such as PFOB/PFD/PFMCP/PFTBA in the ratio from 1/1/1/1 to 10/10/10/10, respectively; the mix of perfluorocarbons in the emulsion contains from 1% (0.5% vol.) to 100% (50% vol.). As rapidly eliminated perfluorocarbons (C₈-C₁₀) are used: perfluorodecaline (PFD) and perfluorooctylbromide (PFOB). As slowly eliminated (C₁₁-C₁₂) perfluorocarbons are used: perfluoromethylcyclohexylpiperidine (PFMCP) and perfluorotributylamine (PFTBA). As emulsifying agent a non-ionogenic block-co-polymer - proxanol-268 having molecular weight 6-12 thousand Da in the concentration from 0.2% to 20% is used.

The formulation of perfluorocarbon gas transferring emulsion contains: dispersion of particles from 1 to 100 nanometers at the quantity 88%, from 100 to 200 nanometers - 12%, from there the quantity of 200 nm particles is not more than 0.1%, average emulsion particles size is 30-80 nanometers.

The composition of perfluorocarbon gas transferring emulsion includes physiologically acceptable electrolyte solution: NaCl, KCl, MgCl₂, NaHCO₃, NaH₂PO₄, D-glucose.

The composition of perfluorocarbon gas transferring emulsion, first variant, contains electrolytes: NaCl - 6.0-9.0 g/l; KCl-0.39-0.45 g/l; MgCl₂ - 0.19 - 0.25 g/1; NaHCO₃ - 0.65-0.70 g/l; NaH₂PO₄ - 0.20-0.260 g/l; D-glucose - 2.0-2.5 g/l (in the finished dosage form).

The composition of perfluorocarbon gas transferring emulsion, second variant, contains a simplified variant of electrolytes: NaCl - 6.0-9.0 g/l; NaH₂PO₄- 0.20-0.60 g/l (in the finished dosage form).

The composition of perfluorocarbon gas transferring emulsion, third and the most favourable variant, contains: rapidly eliminated perfluorocarbon - perfluorodecaline (PFD) - 130-140 g/l; slowly eliminated perfluorocarbon - perfluoromethylcyclohexylpiperidine (PFMCP) - 65-70 g/l; proxanol-268 - 40-42 g/l; electrolytes NaCl - 6.0-9.0 g/l; KCl-0.39-0.45 g/l; MgCl₂ - 0.19 - 0.25 g/l; NaHCO₃ - 0.65-0.70 g/l; NaH₂PO₄ - 0.20-0.260 g/l; D-glucose - 2.0-2.5 g/l (in the finished dosage form).

Composition of perfluorocarbon gas transferring emulsion in the preferred fourth variant contains: rapidly eliminated perfluorocarbon - perfluorodecaline (PFD) - 130-140 g/l: slowly eliminated perfluorocarbon - perfluorocyclohexylpiperidine (PFMCP) - 65-70 g/l; proxanol-268 - 40-42 g/l; simplified variant of electrolytes: NaCl - 6.0-9.0 g/l; NaH₂PO₄ - 0.20-0.60 g/l (in the finished dosage form).

Composition of perfluorocarbon gas transferring emulsion in the preferred fifth variant contains: rapidly eliminated perfluorocarbon - perfluorodecaline (PFD) - 65-70 g/l: slowly eliminated perfluorocarbon - perfluorocyclohexylpiperidine (PFMCP) -32.5-35 g/l; proxanol-268 - 20-22 g/l; electrolytes: NaCl - 6.0-9.0 g/l; KCl - 0.39-0.45 g/l; MgCl₂ - 0.19-0.25 g/l; NaHCO₃ - 0.65-0.70 g/l; NaH₂PO₄ - 0.20-0.60 g/l (in the finished dosage form).

Composition of perfluorocarbon gas transferring emulsion in the preferred sixths variant contains: rapidly eliminated perfluorocarbon - perfluorodecaline (PFD) - 65-70 g/l: slowly eliminated perfluorocarbon - perfluorocyclohexylpiperidine (PFMCP) -32.5-35 g/l; proxanol-268 - 20-22 g/l; simplified variant of electrolytes: NaCl - 6.0-9.0 g/l; NaH₂PO₄ - 0.20-0.60 g/l (in the finished dosage form).

Probable composition based on perfluorocarbon gas transferring emulsion consists of four different perfluorocarbons having chemical and physical properties, which change from extremely lypophylic (PFOB) to sharply expressed lipophobic (PFTBA).

Probable composition based on perfluorocarbon gas transferring emulsion has broad band of different physical and chemical and medical-biological properties due to lipophylic-lipophobic properties of compound perfluorocarbons: rapidly eliminated (PFOB, PFD) and slowly eliminated (PFMCP, PFTBA), which:
Increases effectiveness and quality of emulsion; decreases intramolecular diffusion; increases emulsion stability; improves emulsifying; decreases emulsion toxicity; decreases frequency of adverse reactions of emulsion; increases emulsion shelf-lives; extends spheres of administration.

Probable composition based on perfluorocarbon gas transferring emulsion can be used as synthetic perfluor carbonic blood substitution medium for substances for treatment of different diseases.

Probable composition based on perfluorocarbon gas transferring emulsion can be used as synthetic perfusion agents.

Probable composition based on perfluorocarbon gas transferring emulsion can be used as synthetic X-rays contrast agents.

Probable composition based on perfluorocarbon gas transferring emulsion can be used for external use as a base for water-soluble therapeutic ointments or creams for application, irrigation, moisturising of slowly treated wounds, ulcers and other external diseases.

Proposed composition based on perfluorocarbon gas transferring emulsion can be used for external use as a base for cosmetic water-soluble ointments and skin care creams.

Proposed composition based on perfluorocarbon gas transferring emulsion has a 2.5 higher concentration of perfluorocarbons than analogous compositions, that fact increases oxygen capacity of the preparation significantly.

So, all the presented positive factors approve more effective and safe use of proposed perfluorocarbon gas transferring emulsion in medicine and biology and extend their areas of application.

The second determined problem has the solution through known method of production of perfluorocarbon gas transferring emulsion which includes obtaining of emulsions by the mixing of some quantity of perfluorocarbons with emulsifying agent, multiple homogenization of so obtained mix with high pressure homogenizer, as per the invention, all the process of preparation consists of:
jet-passing of perfluorocarbon mix through water solution of emulsifying agent to obtain required components ratio, at least, in two high pressure homogenizer's extrusion devices, passing the emulsion step by step through essential and additive extrusion devices of the homogenizer till 4-5 times, pass the emulsion through buffer container, which is between two extrusion devices of the homogenizer and is set for pressure balance between devices, passing of emulsion through basic extrusion device gives homogenization pressure increase in 2-3 times in comparison with the pressure in the additional extrusion device, passing of emulsion through the buffer and basic containers gives carbonic acid gas providing. For emulsion production pressure in two extrusion devices should be from 120 - 1500 kg/cm² and the mix of two perfluorocarbons: rapidly eliminated (C₈-C₁₀) and/or slowly eliminated (C₁₁-C₁₂), such as perfluorodecaline (PFD)/perfluoromethylcyclohexylpiperidine (PFMCP), or PFD/perfluorotributylamine (PFTBA), or perfluorooctylbromide (PFOB)/PFMCP, or PFOB/PFTBA, or PFOB/PFD, or PFMCP/PFTBA in the ratio from 1/1 to 10/10, respectively; or the mix of three perfluorocarbons: two rapidly eliminated (C₈-C₁₀) and one slowly eliminated (C₁₁-C₁₂), or one rapidly eliminated (C₈-C₁₀) and two slowly eliminated (C₁₁-C₁₂), such as PFOB/PFD/PFMCP, or PFOB/PFD/PFTBA, or PFOB/PFMCP/PFTBA, or PFD/PFMCP/PFTBA in the ratio form 1/1/1 to 10/10/10, respectively; or the mix of four perfluorocarbons: two rapidly eliminated (C₈-C₁₀) and two slowly eliminated (C₁₁-C₁₂), such as PFOB/PFD/PFMCP/PFTBA in the ratio from 1/1/1/1 to 10/10/10/10, respectively; add to the obtained mix required quantity of electrolytes and filtrate the emulsion dosage form and sterilize it by the dynamic ultrafiltration. After sterilizing by dynamic ultrafiltration emulsion has maximal particle size of 200 nanometers with average particle size 30-80 nm.

### Short description of the figure

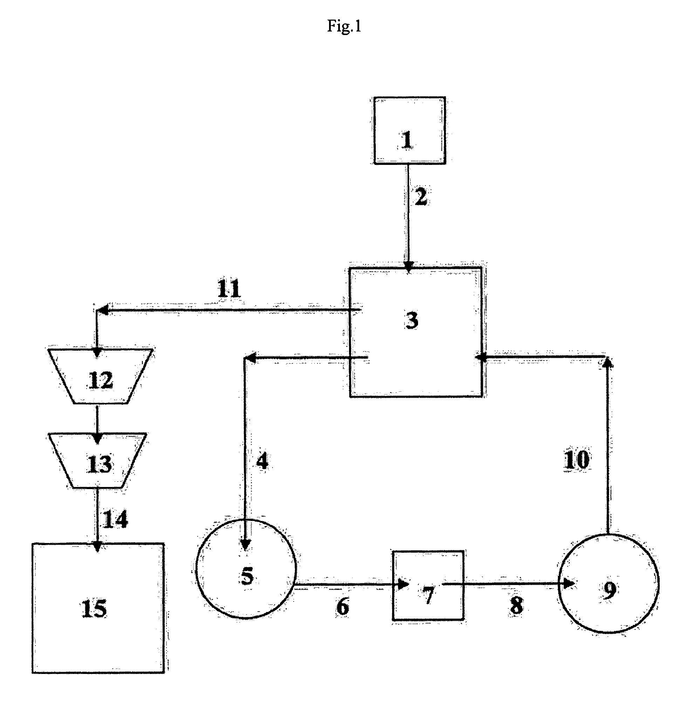

The invention is described by the schema (fig. 1) of the process of method of perfluorocarbon gas transferring emulsion production. On this figure the homogenizing system is reflected. It consists of:
Upper (perfluorocarbonic) container 1 for the perfluorocarbons mix; piping 2, which joints container 1 to the lower (basic) container 3; piping 4, which joints containers 1 and 3 to the basic extrusion device of the homogenizer 5; ducts 6 and 8, which joint basic extrusion device 5 and buffer container 7 and additional extrusion device 9; piping 10, which joints basic container 3; piping 11, which joints filtration 12 and sterilization 13 systems; piping 14, which joints filler container 15.

Perfluorocarbon gas transferring emulsion production method is realized as below:
For pre-emulsion obtaining (micron size) perfluorocarbonic mix comes by stream from the upper (perfluorocarbonic) container 1 by piping 2 to the lower (basic) container 3 with emulsifying agent - proxanol-268 and through piping 4 comes to the basic extrusion device 5 of the high pressure homogenizer, where the high "percussive" pressure has to be created, from 360 to 1500 kg/cm². After that the pre-emulsion comes from the basic extrusion device 5 through the piping 6 to the buffer container 7, where pressure between extrusion devices is equalized, from the container 7 through the piping 8 this emulsion comes to the additional extrusion device 9 of the high pressure homogenizer, in which homogenization pressure is 2-3 times lower than in the basic extrusion device. From the additional extrusion device the pre-emulsion comes through the piping 10 to the basic container 3. The first cycle of pre-emulsion preparation is finished: pre-emulsion instead of one homogenization (decomposition to nano-sizes) in the basic extrusion device obtains additional (second) equal processing ("decomposition") in the additional extrusion device, which decreases preparation time of nanosized emulsion. After the second emulgation cycle, which is completely analogous to the first one, the third one starts etc. till 5 cycles. During all the process of nanoemulsion preparation carbonic acid gas blow has to be produced to the containers and temperature keeping at the level of +15 °C to +60 °C in both extrusion devices. After obtaining of nano-sized perfluorocarbon emulsion, it should be mixed in the basic container 3 with electrolytes for obtaining of ready dosage form. After that emulsion dosage form comes to the filtration 12 and sterilization 13 chambers for filtration and sterilization, after that it comes to the container 15 for filling into the flasks.

Proposed method of preparation of perfluorocarbon gas transferring emulsions allows to create multicomponent emulsions, what improves physical and chemical and medical and biological properties, increases effectiveness of the emulsion.

Proposed method of preparation of perfluorocarbon gas transferring emulsions allows to create highly concentrated emulsions, what increases area of application and quantity of dosage form.

Proposed method of preparation of perfluorocarbon gas transferring emulsions allows to create highly stable emulsions, which increases quality and level of its safe application.

Proposed method of preparation of perfluorocarbon gas transferring emulsions allows to create emulsions which stand sterilization, which improves quantity and level of safe application.

Proposed method of preparation of perfluorocarbon gas transferring emulsions allows to create nano-sized emulsions of 30-80 nanometers, what improves quality and effectiveness of application, decreases side effects quantity.

Proposed method of preparation of perfluorocarbon gas transferring emulsions allows to shorten the time of emulsion production ∼ 2 times due to the decrease of emulsifying cycles in comparison with analogous methods from 9-12 to 4-5, that increases quantity of produced dosage form and improves quality and safety levels in application of the emulsion.

In the suggested method of production for perfluorocarbonic gas transferring emulsions, besides the abovementioned factors, there is another significant advantage: in the declared method an operator, preparing emulsion, is spent two-times less time in the sterilization box than in the method-analogue. A long-term stay in the box can negatively effect the operator activity, and his further mistakes during technological operations. Besides, saving of human and energy- raw material resources occurs.

The developed method can be confidently used not only in laboratory conditions, but also in the industrial production of perfluorocarbonic gas transferring emulsions to obtain commercial batches of synthetic perfluorocarbonic blood substituting preparations and other therapeutic products.

### Detailed description of the invention.

### Composition (formulation) production.

### Example 1. Production of composition of 1% (0,5 vol. %) emulsion of perfluorocarbons PFD/PFMCP.

Mix a composition based on perfluorocarbonic mixture of PFD/PFMCP (ratio 2:1) in amount of 20 ml, contained weightings (liquid) 26 g of PFD and 13 g of PFMCP, with 80 ml of aqueous solution of proxanol-268, contained 8 gram of the dry emulsifier. Specific gravity of perfluorocarbons is ∼ 2. Pass the obtained mixture of perfluorocarbons and proxanol-268 through two extrusion devices of high-pressure homogenizer. After it add to the obtained perfluorocarbonic emulsion a simplified variant of electrolytic solution till the appropriate concentration.

The final formulation (medicinal form) of perfluorocarbonic emulsion has a composition as follows: PFD/PFMCP (ratio 2/1) - 1% (0,5 vol. %); PFD - 6,5 g/l; PFMCP - 3,25 g/l; proxanol-268 - 2,0 g/l; NaCl - 6,0 g/l; MaH₂PO₄ - 0,20 g/l.

The obtained perfluorocarbonic formulation (composition) can be used as a blood substituting drug product at intravenous and intraarterial infusion, peroral, intracavity, oral and topical use.

### Example 2. Production of formulation of 20% (10 vol. %) emulsion of perfluorocarbons PFD/PFMCP.

Mix a composition based on perfluorocarbonic mixture of PFD/PFMCP (ratio 2:1) in amount of 200 ml, contained weightings (liquid) 260 g of PFD and 130 g of PFMCP, with 800 ml of aqueous solution of proxanol-268, contained 80 gram of the dry emulsifier. Pass the obtained mixture of perfluorocarbons and proxanol-268 through two extrusion devices of high-pressure homogenizer. After it add to the obtained perfluorocarbonic emulsion an electrolytic solution till the appropriate concentration.

The final formulation (medicinal form) of perfluorocarbonic emulsion has a composition as follows: PFD/PFMCP (ratio 2/1) - 20% (10 vol.%); PFD - 130 g/l; PFMCP - 65 g/l; proxanol-268 - 40 g/l; NaCl - 6,0 g/l; KCl - 0,39 g/l; MgCl₂ - 0,19 g/l; NaHCO₃ - 0,65 g/l; NaH₂PO₄ - 0,20 g/l; D-glucose - 2,0 g/l.

The obtained perfluorocarbonic formulation (composition) can be used as a blood substituting drug product at intravenous and intraarterial infusion, peroral, intracavity, oral and topical use.

### Example 3. Production of formulation of 30% (15 vol.%) emulsion of perfluorocarbons PFD/PFMCP.

Mix a composition based on perfluorocarbonic mixture of PFD/PFMCP (ratio 2:1) in amount of 300 ml, contained weightings (liquid) 400 g of PFD and 200 g of PFMCP, with 700 ml of aqueous solution of proxanol-268, contained 120 gram of dry emulsifier. Pass the obtained mixture of perfluorocarbons and proxanol-268 through two extrusion devices of high-pressure homogenizer. After it add to the obtained perfluorocarbonic emulsion a simplified variant of electrolytic solution till the appropriate concentration.

The final formulation (medicinal form) of perfluorocarbonic emulsion has a composition as follows: PFD/PFMCP (ratio 2/1) - 30% (15 vol.%); PFD - 200 g/l; PFMCP - 100 g/l; proxanol-268 - 60 g/l; NaCl - 6,0 g/l; NaH₂PO₄ - 0,20 g/l.

The obtained perfluorocarbonic formulation (composition) can be used as a blood substituting drug product at intravenous and intraarterial infusion, peroral, intracavity, oral and topical use.

### Example 4. Production of composition of 80% (40 vol.%) emulsion of perfluorocarbons PFOB/PFD/PFMCP/PFTBA

Composition on the base of perfluorocarbonic mixture of PFOB/PFD/PFMCP/PFTBA (ratio 10/1/1/1) in amount of 400 ml, contained weightings (liquid) PFOB/PFD/PFMCP/PFTBA was made, respectively, in 617/61/61/61 grams. Mix a mixture of perfluorocarbons with 600 ml of aqueous solution of proxanol-268, contained 160 gram of dried emulsifier. Pass the obtained mixture of perfluorocarbons and proxanol-268 through two extrusion devices high-pressure homogenizer. After it add electrolytes up to appropriate concentration into the obtained perfluorocarbonic emulsion.

The final formulation (medicinal form) of perfluorocarbonic emulsion has a composition as follows: PFOB/PFD/PFMCP/PFTBA (ratio 10/1/1/1) - 80% (40 vol.%); PFOB - 617 g/l; PFD - 61 g/l; PFMCP - 61 g/l; PFTBA - 61 g/l; proxanol-268 - 160 g/l; NaCl - 6,0 g/l; NaH₂PO₄ - 0,20 g/l. The obtained perfluorocarbonic composition can be used as a radioopaque contrast agent at intravenous and intraarterial infusion, peroral, intracavity and oral use.

### Example 5. Production of composition of 100% (50 vol.%) emulsion of perfluorocarbons PFD/PFMCP.

Mix a composition based on perfluorocarbonic mixture of PFD/PFMCP (ratio 2:1) in amount of 500 ml, contained weightings (liquid) 666 g of PFD and 333 g of PFMCP, with 500 ml of aqueous solution of proxanol-268, contained 200 gram of dry emulsifier. The specific gravity of perfluorocarbons is - 2. Pass the obtained mixture of perfluorocarbons and proxanol-268 through two extrusion devices of high-pressure homogenizer. After it add to the obtained perfluorocarbonic emulsion a simplified variant of electrolytic solution till the appropriate concentration.

The final formulation (medicinal form) of perfluorocarbonic emulsion has a composition as follows: PFD/PFMCP (ratio 2/1) - 100% (50 vol.%); PFD - 666 g/l; PFMCP - 333 g/l; proxanol-268 - 200 g/l; NaCl - 6,0 g/l; NaH₂PO₄ - 0,20 g/l.

The obtained perfluorocarbonic composition can be used at topical use as an ointment or cream for medical and cosmetic purposes.

### Detailed description of the invention.

### Method of production.

### Example 6. Method of production of 1% (0,5 vol.%) perfluorocarbonic emulsion of PFD/PFMCP.

Mix a perfluorocarbonic mixture of PFD/PFMCP in amount of 200 ml, in ratio of PFD/PFMCP 2/1, contained weightings (liquid) comprising 260g of PFD and 130 g of PFMCP, with 800 ml of proxanol-268 solution, contained 80 grams of the dried emulsifier. A specific gravity of perfluorocarbons is - 2.

To produce pre-emulsion (micron size) a mixture of perfluorocarbons streams from the upper (perfluorocarbonic) capacity 1 by tubing (pipeline) 2 into a lower (base) capacity 3 with emulsifying agent - proxanol-268 and, through a tubing 4 a pre-emulsion enters into a basic extrusion device 5 of a high-pressure homogenizer, where a high "impact" pressure from 360 to 1500 kg/cm is fixed.

Then from the basic extrusion device 5, pre-emulsion enters into a tubing 6 into a buffer capacity 7, equalized pressure between extrusion devices, emulsion enters from capacity 7 through tubing 8 into additional extrusion device 9 of the high-pressure homogenizer, in which the homogenization pressure is 2-3 times lower than in the basic extrusion device. From the additional extrusion device pre-emulsion enters into capacity 3 through tubing 10. The first cycle of the pre-emulsion production is closed: instead of one homogenization treatment ("desintegration" to nanosize) in the basic extrusion device the pre-emulsion is exposed to the additional (second) equal treatment ("desintegration") in the additional extrusion device, which is 2 times reducing preparation time of the nanosized emulsion. After the second emulsification cycle, which is fully analogous to the first cycle, except the homogenization pressure, the third cycle emulsification started etc. till 5 cycles. Within the whole process of production of nanoemulsion occurred the feeding (pumping up) of carbon dioxide and maintained temperature from +15 C⁰ to +60 C⁰ in the capacities of both extrusion devices. After production of nanosized perfluorocarbonic emulsion, the latter is mixed in the basic capacity 3 with electrolytes to obtain the final drug product. Then the medicinal form (drug product) of emulsion enters in the filtration 12 and sterilizing chambers 13, for filtration and/or sterilization, with consequent entering of the final medicinal form of emulsion into capacity 15 to be filled in vials.

The final formulation (medicinal form) of perfluorocarbonic emulsion has a composition as follows: PFD/PFMCP (ratio 2/1) - 1% (0,5 vol.%); PFD - 6,5 g/l; PFMCP - 3,25 g/l; proxanol-268 - 2,0 g/l; NaCl - 6,0 g/l; NaH₂PO₄ - 0,20 g/l. Amount of emulsification cycles is - 5, at the average particle size - 60 nanometers. After 3-times freezing / unfreezing the emulsion is stable, the average particle size is 80 nm. Reactogenity of emulsion is not more than 5%.

### Example 7. Method of production of 20% (10 vol.%) perfluorocarbonic emulsion of PFD/PFMCP.

Perfluorocarbonic mixture PFD/PFMCP in amount of 200 ml, in ratio of PFD/PFMCP 2/1, contained weightings (liquid), comprising 260g of PFD and 130 g of PFMCP, has been mixed with 800 ml of proxanol-268 solution, contained 80 grams of the dried emulsifier. A specific gravity of perfluorocarbons is - 2. After that treat the obtained mixture of perfluorocarbons and proxanol in the same way as described in Example 1. Add electrolytes till required concentration into the obtained emulsion.

The final formulation (medicinal form) of perfluorocarbonic emulsion has a composition as follows: PFD/PFMCP (ratio 2/1) - 20% (10 vol.%); PFD - 130 g/l; PFMCP - 65 g/l; proxanol-268 - 40 g/l; NaCl - 6,0 g/l; KCl - 0,39 g/l; MgCl₂ - 0,19 g/l; NaHCO₃ - 0,65 g/l; NaH₂PO₄ - 0,20 g/l; D-glucose - 2,0 g/l. Amount of emulsification cycles is - 5, at the average particle size - 60 nanometers. After 3-times freezing / unfreezing the emulsion is stable, the average particle size is 80 nm. Reactogenity of emulsion is not more than 6%.

### Example 8. Method of production of 30% (15 vol.%) perfluorocarbonic emulsion of PFD/PFMCP.

Perfluorocarbonic mixture PFD/PFMCP in amount of 300 ml, in ratio of PFD/PFMCP 2:1, contained weightings (liquid), comprising 400g of PFD and 200 g of PFMCP, has been mixed with 700 ml of proxanol-268 solution, contained 120 grams of the dried emulsifier. A specific gravity of perfluorocarbons is - 2. After that, treat the obtained mixture of perfluorocarbons and proxanol in the same way as described in Example 1. Add electrolytes till required concentration in to the obtained emulsion.

The final formulation (medicinal form) of perfluorocarbonic emulsion has a composition as follows: PFD/PFMCP (ratio 2/1) - 30% (15 vol.%); PFD - 200 g/l; PFMCP - 100 g/l; proxanol-268 - 60 g/l; NaCl - 6,0 g/l; NaH₂PO₄ - 0,20 g/l. Amount of emulsification cycles is - 5, at the average particle size - 70 nanometers. After 3-times freezing / unfreezing the emulsion is stable, the average particle size is 80 nm. Reactogenity of emulsion is not more than 6%.

### Example 9. Method of production of 80% (40 vol.%) perfluorocarbonic emulsion of PFOB/PFD/PFMCP/PFTBA.

Perfluorocarbonic mixture of PFOB/PFD/PFMCP/PFTBA in amount of 400 ml, (in ratio of 2:1), contained weightings (liquid) of PFOB/PFD/PFMCP/PFTBA, comprising respectively, 617/61/61/61 grams, has been mixed with 600 ml of proxanol-268 solution, contained 160 grams of the dried emulsifier. A specific gravity of perfluorocarbons is - 2. After that treat the obtained mixture of perfluorocarbons and proxanol in the same way as described in Example 1. Add electrolytes till required concentration in to the obtained emulsion.

The final formulation (medicinal form) of perfluorocarbonic emulsion has a composition as follows: PFOB/PFD/PFMCP/PFTBA (ratio 10/1/1/1) - 80% (40 vol.%); PFOB - 617 g/l; PFD - 61 g/l; PFMCP - 61 g/l; PFTBA -61 g/l; proxanol-268 - 160 g/l; NaCl - 6,0 g/l; NaH₂PO₄ - 0,20 g/l. Amount of emulsification cycles is - 5, at the average particle size - 70 nanometers. After 3-times freezing / unfreezing the emulsion is stable, the average particle size is 80 nm.

### Example 10. Method of production of 100% (50 vol.%) perfluorocarbonic emulsion of PFD/PFMCP.

Perfluorocarbonic mixture of PFD/PFMCP (ratio 2:1) in amount of 500 ml, contained weightings (liquid) of 666 g of PFD and 333 g of PFMCP, has been mixed with 500 ml of aqueous solution of proxanol-268, contained 200 grams of the dried emulsifier. A specific gravity of perfluorocarbons - 2. After that treat the obtained mixture of perfluorocarbons and proxanol in the same way as described in Example 1. Add electrolytes till required concentration in to the obtained emulsion.

The final formulation (medicinal form) of perfluorocarbonic emulsion has a composition as follows: PFD/PFMCP (ratio 2/1) - 100% (50 vol.%); PFD - 666 g/l; PFMCP - 333 g/l; proxanol-268 - 200 g/l; NaCl - 6,0 g/l; NaH₂PO₄ - 0,20 g/l. Amount of emulsification cycles is - 5, at the average particle size - 70 nanometers. After 3-times freezing / unfreezing the emulsion is stable, the average particle size is 80 nm.

**Table 1.**

| Some physical-chemical and medicobiological characteristics of perfluorocarbonic gas transferring emulsions | | | | |
|---|---|---|---|---|
| Parameters | Composition-analogue Fluosol-DA | Composition-analogue 1, patent No. 2162692 | Composition-analogue 2, patent No. 2199311 | Claimed composition: |
| | *Particle distribution (%)* | | | |
| Less than 0,1 µm | 39,2 | 87,1 | 87,1 | 88,0 |
| 0,1 - 0,2 µm | 53,0 | 12,6 | 12,9 | 12,0 |
| 0,2 - 0,3 µm | 5,9 | 0,3 | - | - |
| 0,3 - 0,4 µm | 1,5 | - | - | - |
| 0,4 - 0,5 µm | 0,4 | - | - | - |

| | *Other parameters* | | | |
|---|---|---|---|---|
| Sterilized | No | No | Sterilized | Sterilized |
| Side effects (%) | not known | 10 | 6 | 5-6 |
| Amount of homogenization cycles | not known | 12 times | 9 times | 5 times |
| Content of PFOC | 20% | to 20% | to 30% | to 100% |
| Preparation time for 10 litres of emulsion | not known | 4 hours | 3 hours | 1,5 hours |
| Amount of extruders (pcs.) | 1 | 1 | 1 | 2 |

## Claims

1. Composition of perfluorocarbon and gas transferring emulsion for medicobiological purposes: composition, method of production and preparation for treatment (variants), *comprising*
perfluorocarbons, emulsifying agent and electrolytic solution, ***characterized in* that** it contains:
- a mixture of two perfluorocarbons: fast eliminated (C₈-C₁₀) and/or slowly eliminated (C₁₁-C₁₂), as perfluorodecaline (PFD) /perfluoromethylcyclohexylpiperidine (PFMCP), or PFD/perfluorotributylamine (PFTBA), or perfluorooctylbromide (PFOB)/PFMCP, or PFOB/PFTBA, or PFOB/PFD, or PFMCP/PFTBA in ratio from 1/1 to 10/10, respectively;
- or a mixture of three perfluorocarbons: two of them are fast eliminated (C₈-C₁₀) and one is slowly eliminated (C₁₁-C₁₂), or one fast eliminated (C₈-C₁₀) and two slowly eliminated (C₁₁-C₁₂), as PFOB/PFD/PFMCP, or PFOB/PFD/PFTBA, or PFOB/PFMCP/PFTBA, or PFD/PFMCP/PFTBA in ratio from 1/1/1 to 10/10/10, respectively;
- or a mixture of four perfluorocarbons: two of them are fast eliminated (C₈-C₁₀) and the rest two are slowly eliminated (C₁₁-C₁₂) PFOB/PFD/PFMCP/PFTBA in ratio from 1/1/1/1 to 10/10/10/10, respectively,

2. Composition of perfluorocarbon gas transferring emulsion according to claim 1, ***characterized in* that** as the fast eliminated perfluorocarbons (C₈-C₁₀) are used: perfluorodecaline (PFD) and perfluorooctylbromide (PFOB).

3. Composition of perfluorocarbon gas transferring emulsion according to claim 1, ***characterized in* that** as slowly eliminated (C₁₁-C₁₂) perfluorocarbons are used: perfluoromethylcyclohexylpiperidine (PFMCP) and perfluorotributylamine (PFTBA).

4. Composition of perfluorocarbon gas transferring emulsion according to claim *1,* ***characterized in* that** the amount of perfluorocarbons in emulsion is from 1% (0,5 vol.%) to 100% (50 vol.%).

5. Composition of perfluorocarbon gas transferring emulsion according to claim 1, ***characterized in* that** the particle dispersion from 1 to 100 nanometers amounts to - 80-90%, from 100 to 200 nanometers - 10-20%, when the average particle size of emulsion is 30-80 nanometers.

6. Composition of perfluorocarbon gas transferring emulsion according to claim 1, ***characterized in* that** nonionic block copolymer of ethylene and propylene oxide - proxanol-268 from 0,2% to 20% with the molecular weight 6-12 thousand Da is used as emulsifying agent.

7. Composition of perfluorocarbon gas transferring emulsion according to claim 1, ***characterized in* that** physiologically acceptable electrolytic solution includes: NaCl, KCl, MgCl₂, NaHCO₃, NaH₂PO₄, D-glucose.

8. Composition of perfluorocarbon gas transferring emulsion according to claim 1, ***characterized in* that** in the first variant electrolytic solution contains: NaCl - 6,0-9,0 g/l; KCl - 0,39-0,45 g/l; MgCl₂ - 0,19-0,25 g/l; NaHCO₃ - 0,65-0,70 g/l; NaH₂PO₄ - 0,20-0,60 g/l; D-glucose - 2,0-2,5 g/l (in the final dosage form).

9. Composition of perfluorocarbon gas transferring emulsion according to claim 1, ***characterized in* that** in the second variant electrolytic solution contains: NaCl - 6,0-9,0 g/l; NaH₂PO₄ - 0,20-0,60 g/l (in the final dosage form).

10. Composition of perfluorocarbon gas transferring emulsion according to claim 1, ***characterized in* that** in the preferred third variant it contains: fast eliminated perfluorocarbon - perfluorodecaline (PFD) - 130-140 g/l; slowly eliminated perfluorocarbon - perfluoromethylcyclohexylpiperidine (PFMCP) - 65-70 g/l; proxanol-268 - 40-42 g/l; electrolytic solution: NaCl - 6,0-9,0 g/l; KCl - 0,39-0,45 g/l; MgCl₂ - 0,19-0,25 g/l; NaHCO₃ - 0,65-0,70 g/l; NaH₂PO₄ - 0,20-0,60 g/l; D-glucose -2,0-2,5 g/l (in the final dosage form).

11. Composition of perfluorocarbon gas transferring emulsion according to claim 1, ***characterized in* that** in the preferred fifth variant it contains: the fast eliminated perfluorocarbon - perfluorodecaline (PFD) - 130-140 g/l, the slowly eliminated perfluorocarbon - perfluoromethylcyclohexylpiperidine (PFMCP) - 65-70 g/l, proxanol-268 - 40-42 g/l, electrolytic solution : NaCl - 6,0-9,0 g/l; NaH₂PO₄ - 0,20-0,60 g/l (in the final dosage form).

12. Composition of perfluorocarbon gas transferring emulsion according to claim 1, ***characterized in* that** in the preferred forth variant it contains: the fast eliminated perfluorocarbon - perfluorodecaline (PFD) - 65-70 g/l; the slowly eliminated perfluorocarbon - perfluoromethylcyclohexylpiperidine (PFMCP) - 32,5-35 g/l; proxanol-268 - 20-22 g/l; electrolytic solution : NaCl - 6,0-9,0 g/l; KCl - 0,39-0,45 g/l; MgCl₂ - 0,19-0,25 g/l; NaHCO₃ - 0,65-0,70 g/l; NaH₂PO₄ - 0,20-0,60 g/l; D-glucose - 2,0-2,5 g/l (in the final dosage form).

13. Composition of perfluorocarbon gas transferring emulsion according to claim 1, ***characterized in* that** in the preferred sixth variant it contains: the fast eliminated perfluorocarbon -perfluorodecaline (PFD) - 65-70 g/l, the slowly eliminated perfluorocarbon - perfluoromethylcyclohexylpiperidine (PFMCP) - 32,5-35 g/l, proxanol-268 - 20-22 g/l, electrolytic solution: NaCl - 6,0-9,0 g/l; NaH₂PO₄ - 0,20-0,60 g/l (in the final dosage form).

14. Method of production of perfluorocarbonic gas transferring emulsion for medicobiological purposes, *included:* production of emulsion by mixing of total amount of perfluorocarbons with emulsifying agent, multiple homogenization of the obtained mixture in the homogenizer of high pressure, ***characterized in* that** the whole process of the emulsion preparation is carried out:
at least, in two extrusion high-pressure devices, passing the emulsion sequentially through the basic and additional extrusion devices (extruders), by passing the emulsion through the buffer capacity, located between two extruders and assigned for pressure equalization between the devices, passing the emulsion through the basic extruder, increase the homogenization pressure 2-3 times in comparison with pressure in the additional extruder, passing the emulsion through the buffer capacity, carry out input (delivery) of carbon-dioxide gas.

15. Method of production of perfluorocarbonic gas transferring emulsion according to claim 14, ***characterized in* that** the emulsion is produced by passing of the stream of perfluorocarbons mixture through aqueous solution of emulsifying agent to obtain required component ratio.

16. Method of production of perfluorocarbonic gas transferring emulsion according to claim 14, ***characterized in* that** the emulsion is produced under the pressure in the both extrusion devices from 120 to 1500 kg/cm².

17. Method of production of perfluorocarbonic gas transferring emulsion according to claim 14, ***characterized in* that** the emulsion is passed sequentially through the basic and additional extrusion devices 4-5 times.

18. Method of production of perfluorocarbonic gas transferring emulsion according to claim 14, ***characterized in* that** to obtain emulsion they use a mixture of two perfluorocarbons: the fast eliminated (C₈-C₁₀) and/or the slowly eliminated (C₁₁-C₁₂), as perfluorodecaline (PFD) /perfluoromethylcyclohexylpiperidine (PFMCP), or PFD/perfluorotributylamine (PFTBA), or perfluorooctylbromide (PFOB)/PFMCP, or PFOB/PFTBA, or PFOB/PFD, or PFMCP/PFTBA in ratio from 1/1 to 10/10, respectively.

19. Method of production of perfluorocarbonic gas transferring emulsion according to claim 14, ***characterized in* that** to obtain emulsion they use mixture of three perfluorocarbons: two fast eliminated (C₈-C₁₀) and one slowly eliminated (C₁₁-C₁₂), or one fast eliminated (C₈-C₁₀) and two slowly eliminated (C₁₁-C₁₂), as PFOB/PFD/PFMCP or PFOB/PFD/PFTBA, or PFOB/PFMCP/PFTBA, or PFD/PFMCP/PFTBA in ratio from 1/1/1 to 10/10/10 respectively.

20. Method of production of perfluorocarbonic gas transferring emulsion according to claim 14, ***characterized in* that** to obtain emulsions they use a mixture of four perfluorocarbons: two fast eliminated (C₈-C₁₀) and two slowly eliminated (C₁₁-C₁₂) PFOB/PFD/PFMCP/PFTBA in ratio from 1/1/1/1 to 10/10/10/10 respectively.

21. Method of production of perfluorocarbonic gas transferring emulsion according to claim 14, ***characterized in* that** in the obtained emulsion they add the appropriate amount of electrolytes and expose the medicinal form of emulsion to filtration and sterilization with the help of dynamic ultrafiltration.

22. Method of production of perfluorocarbonic gas transferring emulsion according to claim 14, ***characterized in* that** medicinal form of emulsion is sterilized by the dynamic ultrafiltration.

23. Method of production of perfluorocarbonic gas transferring emulsion according to claim 14, ***characterized in* that** medicinal form of emulsion after sterilization by the dynamic ultrafiltration has the maximal particle size not more than 200 nm, at the average particle diameter is 30-80 nm.

24. Method of production of perfluorocarbonic gas transferring emulsion ***characterized in* that** it is intended for intravenous, intraarterial, peroral, intracavitary application and before the use it can be diluted from 1,2 to 50 times with any solution or formulation compatible with emulsion.

25. Drug product on the basis of perfluorocarbonic gas transferring emulsion according to claim 24, ***characterized in* that** it is intended for topical use as a curative ointment or cream for wounds, ulcers, and other external diseases, before the use it can be diluted from 1,2 to 50 times with any antiviral, antibacterial, fungicidal solution or colloidal silver or another formulation compatible with emulsion.

26. Product on the base of perfluorocarbonic gas transferring emulsion according to claim 24, ***characterized in* that** it is intended for cosmetic purposes as ointment or cream for skin care, before the use it can be diluted from 1,2 to 50 times with any cosmetic or another solution or formulation compatible with emulsion.
